(19) **Europäisches Patentamt / European Patent Office / Office européen des brevets**

(11) **EP 2 957 916 A1**

(12) **EUROPEAN PATENT APPLICATION**

(43) Date of publication:
**23.12.2015  Bulletin 2015/52**

(51) Int Cl.:
**G01N 35/08** (2006.01)          **G01N 33/14** (2006.01)
**G01N 27/49** (2006.01)          **G01N 27/416** (2006.01)

(21) Application number: **15173211.2**

(22) Date of filing: **22.06.2015**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **20.06.2014  CL 20141657**

(71) Applicant: **Universidad de Santiago de Chile Santiago de Chile (ES)**

(72) Inventor: **CAROLI REZENDE, MARCOS SANTIAGO DE CHILE (CL)**

(74) Representative: **Isern-Jara, Nuria Avda. Diagonal 463 Bis 2° 08036 Barcelona (ES)**

(54) **ELECTROANALYTICAL SYSTEM AND METHOD FOR MEASURING ANALYTES**

(57)    The application is directed to a flow injection analysis (FIA) system based on a modified copper working electrode for the amperometric determination of analytes such as sugar, sulfites, ethanol, etc., whose concentration is important in the quality control of wine. This allows use of a larger number of samples, in time, being faster than techniques based on titration volumes requiring order of microliters, which has not been pretreated, thus avoiding the use of reactive contaminants.

**FIGURA 2B**

**Description**

**Field of the Invention**

The present invention relates to a electroanalitical system and method for measuring analytes whose concentration is important in the quality control of wine, such as sugar, sulfites, ethanol, among others, using a larger number of samples over time.

**Prior Art**

The existing traditional techniques of analysis to measure analytes in wine available in the market are based on volumetric titration measures that require great time, reagents and samples.

Other methods of analysis are based on printed electrodes modified with enzymes are also known, but are not robust and have not been tested or adapted to the respective markets.

In patent literature, in particular EP 0805350 discloses an apparatus and method for measuring the concentration of certain chemical substances in clear solutions, turbid and colored, suspensions or emulsions by two pH micro electrodes connected in series. Sampling was done by microdialysis fiber or dilution in a mixing chamber with buffer systems specially formulated. The reaction of interest can started by chemically immobilized reagents or physically confined in a tube reactor interposed between the two pH electrodes, or by standard mixing techniques. The concentration of an analyte is calculated from the formula:

$$[analito]=FCALx(DeltapH_1-DeltapH_0)$$

where FCAL is an experimentally determined calibration factor and $deltapH_0$, $deltapH_1$ are the difference in pH before and after a given chemical reaction that produces or absorbs protons occurred. Substances such as glucose, fructose, sucrose, lactate, ascorbate, citrate, acetate, malate, $CO_2$, bicarbonate, creatinine, urea, triglycerides, proteins, enzymes (lipases, proteases, cholinesterases, glucose-6-phosphate-dehydrogenase (G6PD), phosphatase alkaline, urease and others) or enzyme-inhibiting substances such as antibiotics and organophosphate pesticides can be quickly detected in body fluids, ultrafiltration of hemodialysis, milk, drinks (wine, beer, vegetable juices), surface and ground water, and cell suspensions and the like.

GB2335985 describes an electrode for electrolytic analysis. The electrode has a copper surface with a carbon cover as diamond of a thickness of 0.01 - 5, Å and preferably close to 0.1 Á. The electrode can be solid copper in the form of sheet or wire, or covered with copper or other metal such as nickel or aluminum. The electrode was used to determine analytes which are reacted, particularly oxidation, in copper electrodes. Is particularly applicable to analysis of hydroxy compounds, especially those that are relatively resistant to electrolytic oxidation, for example, sugars and ethanol, as the diamond-like carbon cover eliminates the need for highly alkaline means, and allows the use of pH close to neutral. The analytical technique is preferably differential pulse voltammetry. The electrode can be used to monitor fermentation processes. The electrodes may also be used in fuel cells that use less basic alkaline electrodes.

CN102323313 discloses a method and device for detecting the time of aging wine. The invention discloses a method and a device for detecting the time of dry wine aging. In the device, a wine conditioning container is available in a heating device; an inlet of the container is formed at the top of the container which accommodates the wine; an outlet formed in the bottom of the container which accommodates the wine; an agitator provided in the bottom of the cavity of the container accommodating the wine; an electrode and a diffuser are arranged in the cavity of the container which accommodates the wine; the electrode is connected to a computer through a parameter analyzer and fixed through a support; the diffuser communicates with an oxygen bottle; and a valve that regulates the pressure and flow meter are arranged in the pipe connecting the diffuser and the oxygen bottle. In the method, the data detected by the electrode is transmitted to the computer and processed to obtain a regression model that corrects and predicts a corresponding year and based on regression model and prediction of year can be judged the prediction correction. By the method and device, the aging time of dry wine can be determined carefully and quickly.

CN102323313 discloses a method and device for detecting the aging time of dry wine. In the device, a container which accommodates the wine is placed in a heating device; an inlet of the container is formed at the top of the container which accommodates the wine; an outlet formed in the bottom of the container which accommodates the wine; an agitator provided in the bottom of the container cavity accommodating the wine; an electrode and a diffuser are arranged in the cavity of the container which accommodates wine; the electrode is connected with a computer via a parameter analyzer and fixed through a support; the diffuser communicates with an oxygen bottle; a valve that regulates the pressure and flow meter is arranged in the pipe connecting the diffuser and the oxygen bottle. In the method, the data detected by the electrode is transmitted to the computer and processed to obtain a regression model that predicts and corrects a corresponding year, and based on the regression model that predicts and corrects, the success or failure and year of the same variety of dry wine with the same brand they can be judged. By the method and device, the aging time of dry wine can be determined carefully and quickly.

CN102879445 discloses a device for detecting the aging time of dry wine.

In the wine container device is placed in a heating device, an inlet is formed on top of the container, and the outlet is formed at the bottom of the container; an agitator provided in the bottom of the cavity of the container, and an electrode and a diffuser are arranged in the cavity of the container; the electrode is connected to a computer through a parametric analyzer and attached through a bracket; The diffuser is connected to an oxygen cylinder; and pressure reducing valve and a flow meter are arranged in a pipe through which the diffuser is connected to the oxygen cylinder. According to the detection device, the data detected by the electrode are sent to the computer for processing in order to obtain a regression model of correction and prediction for the year in which the degree of correction of the prediction is judged on time of dry aging wine of the same brand and variety. The device can carefully and quickly confirm a aging time of dry wine.

CN202177599 discloses a device for detecting the aging time of dry wine. In the device a wine container is placed in a heating device; an inlet formed in the top of the container, and an outlet is formed at the bottom of the container; an agitator provided in the bottom of the cavity of the container, and an electrode and a diffuser are placed in the cavity of the container; the electrode is connected to a computer through a parametric analyzer and fixed by a bracket; the diffuser is connected to an oxygen cylinder and a pressure regulating valve and a flow meter placed in the piping through which the diffuser communicates with the oxygen cylinder. According to the detection device, the data detected by the electrode are sent to the computer for processing in order to obtain a regression model of prediction and correction of the corresponding year based on a model where the degree of correction of the prediction is judged on the aging time of the dry wine of the same brand and variety. The device carefully and quickly confirms the aging time of dry wine.

However, it does not have a system and method for measuring analytes such as sugar, sulfites, ethanol, among others, in wines using a greater number of samples, in time

## BRIEF DESCRIPTION OF THE INVENTION

The present invention allows analysis of larger numbers of samples, in time, and employs a flow injection system and printed copper electrodes modified for detection and quantification of analytes, such as sugars, ethanol and sulphite in wine. The amperometric response of these modified electrodes by voltammetric deposition on its surface copper oxide, in an alkaline medium, is selective for each analyte and processed through a data processing means once calibrated with samples of known concentration, the system allows to determine concentrations of analytes in samples of unknown concentrations.

This method has the advantage of being faster than techniques based on qualifications, requiring small volumes of wine (of the order of microliters), without being necessary to pretreat the sample, without the use of reactive contaminants.

## BRIEF DESCRIPTION OF THE FIGURES

Figure 1        illustrates the commercial printed copper electrode, modified by surface depositing of a copper oxide film, which is selective for the analytes described

Figure 2A       illustrates a continuous aqueous stream is injected through the peristaltic pump (a) through the injection system of the wine sample (b), wherein the aqueous flow which carries the sample contacts a small electrochemical cell with the modified printed electrode (c) which, when connected with the potentiostat (d) generates amperometric calibration curves and read measurements in a data processing device, such as a personal computer.

Figure 2B       illustrates the flow diagram for the system shown in Figure 2A.

Figure 3        illustrates the amperometric current intensity graphs versus time. Each sample is recorded at least twice to obtain an average value of the intensity readings.

Figure 4        illustrates the calibration graph, known from five samples, the average current I, viewed in Figure 3, versus the analyte concentration (sugar).

**Figure 5**      illustrates an example of final reading of glucose concentration, through a software for automated analysis processing

**Figure 6**      illustrates amperometric graphs of the current intensity versus time. Each sample is recorded at least twice to obtain an average value of the intensity readings.

**Figure 7**      illustrates the calibration graph, known from four samples, the average current I, viewed in Figure 6, versus analyte concentration (sugar).

## DETAILED DESCRIPTION OF THE INVENTION

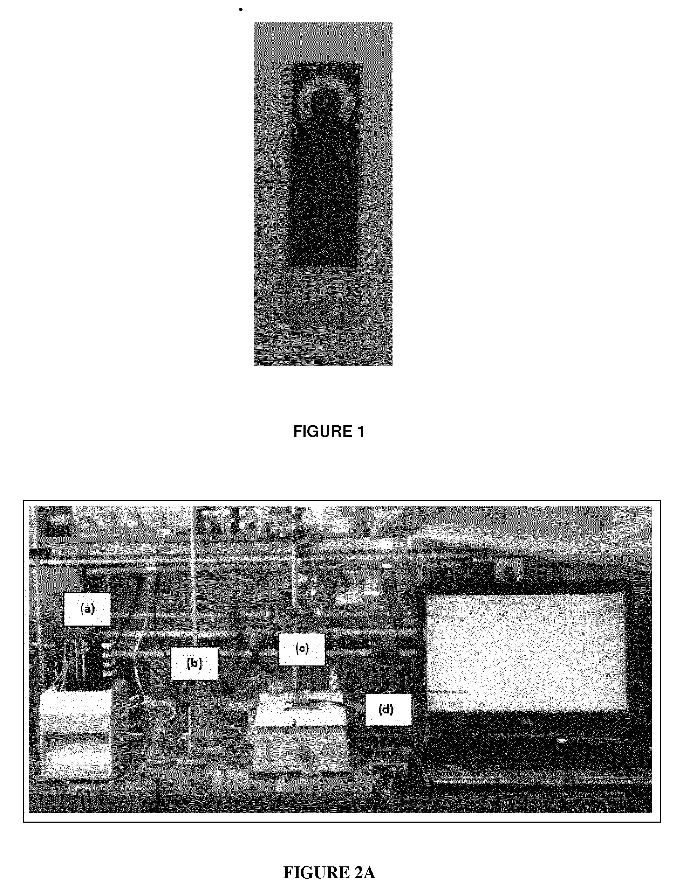

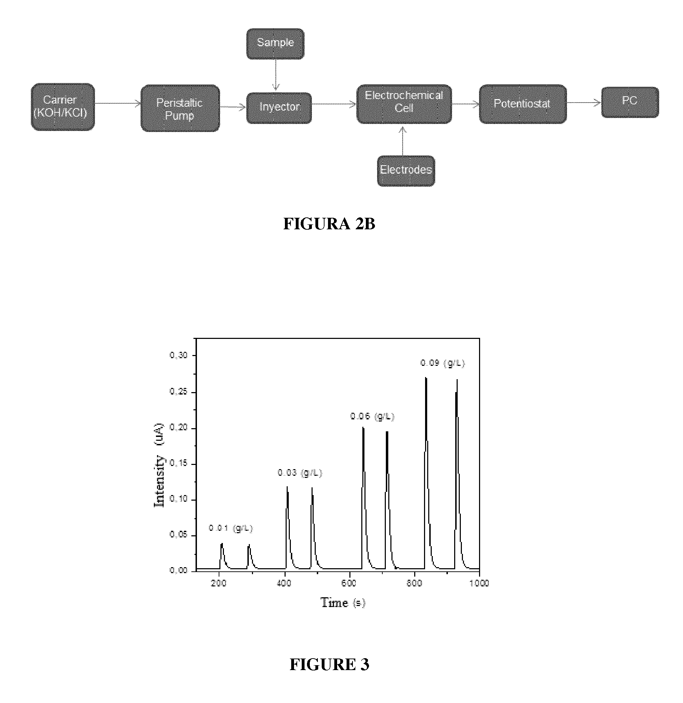

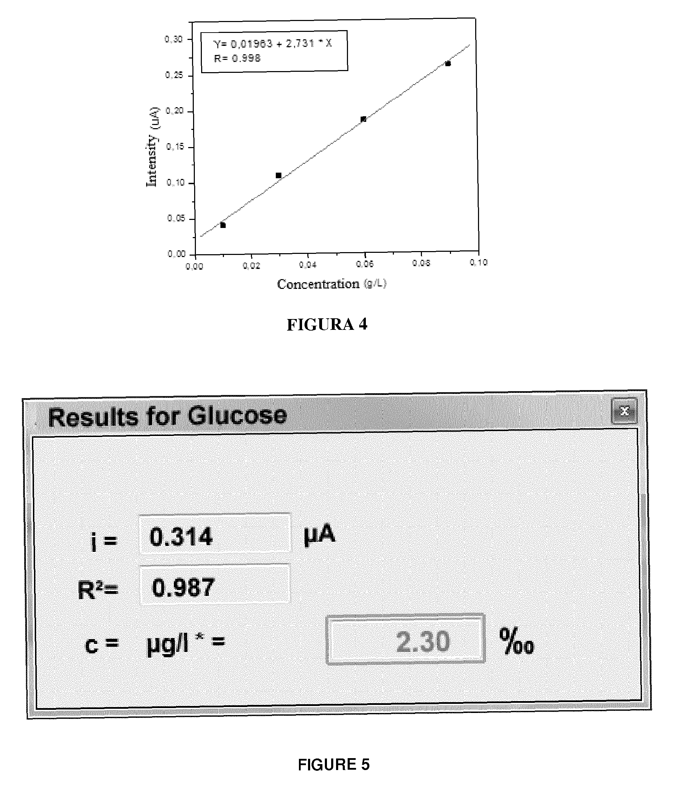

The present invention relates to a electroanalitic method and system for measuring wine analytes, such as sugars, sulfite and ethanol, which uses printed copper electrodes, modified by voltammetric deposition on its surface copper oxide in an alkaline medium (see Figure 1). The modification enables selective amperometric detection of each analyte without interference from other oxidizable compounds in the wine. The system also employs a flow injection method ("flow injection analysis" FIA) to put the basic aqueous solution containing the sample in contact with the electrode, using very small sample volumes (microliters order) of wine. The wine sample is injected into an aqueous continuous flow of NaCl/ NaOH 0.1 M through a analogous flow nozzle to that used in liquid chromatographs. In particular, the continuous flow of aqueous NaCl/NaOH 0.1 M is injected through a driving means (a), for example, a peristaltic pump, passing through the injection system sample (b). The aqueous flow drags the injected sample and contacts a small electrochemical cell modified with the printed copper electrode (c) which, when connected with the potentiostat (d), generates amperometric curves of calibration and processed measurement in a data processing means, such as a computer implemented with a software for processing such data, further wherein the data processing means can receive and process amperometric input data directly or on the site where the sample is taken, or optionally, it can receive and process such data remotely.
The analyte concentration is recorded amperometrically by the printed copper electrode, generating intensity versus time graphs (see Figure 3). Each sample is recorded at least two times, obtaining an average value of the intensity readings. Through prior calibration with samples of known wine concentrations, straight actual recorded intensity versus analyte concentration are plotted, which allow immediate determination of the same analyte concentrations in unknown samples (see Figure 4). The calibration graph is constructed from at least four known samples of average current I read in Figure 3, versus the concentration of the analyte (glucose).
For the prior to system calibration, solutions of glucose and fructose are used, in relative concentration variables. Calibration for red wines, is made using a mixtures of glucose concentration fixed and fructose variable; for white wines, a fixed amount of fructose and glucose concentration variable is used.
The present invention includes software for introduction, calibration and automated sample measurement by an operator, obtaining readings directly in g/L or ppm, from the data processing means (see Figure 5).

Experimental development:

This is illustrated in Figure 3 and a calibration curve was performed for white wine, with samples fixed concentration of fructose (0.01 g/L) and variable glucose, concentrations 0.01; 0.03; 0.06 and 0.09 g/L in 0.1 M NaCl medium. 15 microliters of each solution were injected for detection in a continuous flow of aqueous NaCl/NaOH 0.1 M, and therefore, a signal current (amperometric signal) varying between 0.04 and 0.30 microamperes was obtained.
After injecting the solutions of the calibration curve, 15 microliters of the sample of white wine diluted in 0.1M NaCl medium were injected. These solutions were prepared by taking 300 microliters of wine diluted in 100 mL of 0.1M of NaCl solution (0.3% v/v).

Experimental development (Figure 4):

The curve shown in Figure 4 is obtained by averaging the currents associated with each sample of the calibration curve (Figure 3) and plotting each average value obtained from at least two measurements depending on the concentration in units of g/L. From the curve, we obtain the equation of the line, where the value of X is the concentration of the wine sample.

Experimental development:

The value shown in the green box in Figure 5 corresponds to the concentration of the wine sample provided by the data processing means, this is obtained by replacing the value of the current intensity of the wine sample in the Y component of the equation of the line and calculating the value of X.

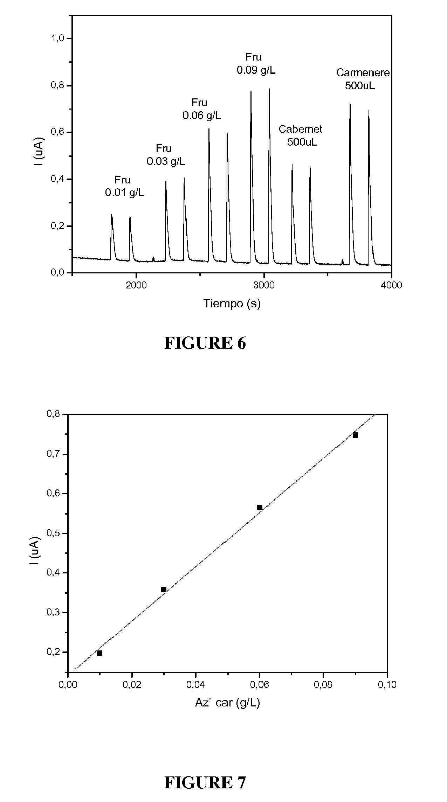

Experimental development (Figure 6):

This is illustrated in Figure 6 and a calibration curve for red wine was performed with samples of fixed glucose concentration (0.01 M) and varying fructose concentrations of 0.01, 0.03, 0.06 and 0 09 g/L, in NaCl medium 0.1 M. 15 microliters of each solution were injected for detection in a continuous flow of aqueous NaCl/NaOH 0.1 M and therefore a signal current is obtained, varying between 0.2 and 0.8 microamperes.

After injecting the solutions of the calibration curve, 15 microliters of two samples of red wines, wine and other wine Cabernet Carmenere they were injected in duplicate, diluted in half 0.1 M NaCl These solutions were prepared by taking 500 microliters wine diluted in 100 mL of 0.1 M NaCl solution (0.5% v/v)

Experimental development (Figure 7);

The line shown in Figure 7 is obtained by averaging the currents associated with each sample of the calibration curve (Figure 6) and plotting each average value obtained from at least two measurements depending on the concentration of sugar units g/L. Of the line obtained can determine the sugar concentration in the unknown samples, calculating the value of X corresponding to the average value of the currents for the sample (Figure 6).

Application example prototype to various samples of wines from different vineyards. Each sample was analyzed once, following described experimental development:

| TIPE WINE (Viña) | SUGAR ANALYZED (g/L) | REFERENCE VALUE HPLC (g/L) | Error (g/L) |
|---|---|---|---|
| Merlot (Morandé) | 1.39 | 1.1 | 0.29 |
| Merlot (Concha y Toro) | 12.89 | 13.1 | 0.21 |
| Sauvignon Blanc (Concha y Toro) | 4.58 | 4.43 | 0.15 |
| Sauvignon Blanc (Morandé) | 6.43 | 6.61 | 0.18 |
| Late Harvest (Morandé) | 31.4 | 30.01 | 1.39 |
| Merlot (Morandé) | 2.19 | 2.08 | 0.11 |
| Cabernet Sauvignon (Morandé) | 8.48 | 8.9 | 0.42 |
| Carmenere (Concha y Toro) | 16.25 | 17.2 | 0.95 |
| Sauvignon Blanc (Morandé) | 6.16 | 6.92 | 0.76 |

The first column values yields sugar concentration in different wines, measured by this electroanalitic system. The second column compares these values with the concentration values measured with a high pressure liquid chromatograph. The last column records the error obtained between the two methods.

**Claims**

**1.** Electroanalytical system to measure analytes of wine, such as sugars, sulfite and ethanol, comprising:

- At least one printed copper electrode, modified by voltammetric deposition on its surface copper oxide, in an alkaline medium;
- A flow injection system to put the wine sample in contact with the modified printed copper electrode using volumes on the order of microliters of sample;
- a flow injector, analogous to the one used in liquid chromatographs, driven by driving means such as a peristaltic pump;
- A small electrochemical cell with the modified printed copper electrode which is connected to a potentiostat and which generates amperometric calibration and measurement curves to be processed in data processor means; and

- data processor means implemented with a software that allows measuring wine samples in an automated way, , providing, in response, direct readings in g/L or ppm, wherein said data processor means can receive and process the input amperometric data ,in a direct way or in the place where the sample is taken, or optionally, can receive and process such data remotely.

2. Electroanalytical method for measurement of analytes of wine, such as sugars, sulfite and ethanol comprising:

- Contacting amounts in the order of microliters from a wine sample, with at least one printed copper electrode, modified by voltammetric deposition on its surface copper oxide in an alkaline medium, using a flow injection system that injects a continuous aqueous flow of NaCl / NaOH 0.1 M, through a flow injector similar to the one used in liquid chromatographs, and preferably driven by a peristaltic pump;

wherein the continuous aqueous flow of NaCl / NaOH 0.1 M passes through a system where the sample is injected, and the sample, which is dissolved in the aqueous flow, is contacted with a small electrochemical cell having the modified printed copper electrode connected with potentiostat for generating amperometric curves for calibration and measurements, and the curves are processed in data processing means, indicating the analyte concentration recorded by the printed electrode, generating intensity versus time graphs;
wherein each wine sample is recorded at least twice to obtain an average value of the intensity readings; and
wherein the data processor means have been implemented with a software that allows automated measurements in wine samples, obtaining direct readings in g / L or ppm, in the data processor means; and

- Building a previous calibration curve with wine samples of known concentrations wherein are drawn straight lines of the registered intensity versus the actual analyte concentration, and the straight lines are then used for immediate determination of the concentrations of the same analyte in samples with unknown concentrations of the analyte,

wherein said calibration curve is constructed from a number of known samples of average intensity current versus analyte concentration; and
where the previous calibration involves the use of glucose + fructose solutions of varying relative concentrations, and for red wines, the calibration is done using mixtures of fixed concentration glucose and variable concentration of fructose, while for white wines, is used a fixed concentration of fructose and a variable concentration of glucose.

3. The method of claim 2 **comprising** constructing a calibration curve for white wine with a fixed concentration of fructose (0.01 M) and a variable concentration of glucose concentrations 0.01; 0.03; 0.06 and 0.09 g / L in 0.1M NaCl medium, injecting 15 microliters of each solution in a NaCl / NaOH 0.1 M flow for detection, and thus obtain a current or amperometric signal.

4. The method of claim 3, further **comprising** injecting 15 microliters of sample of white wine diluted in 0.1M NaCl medium, which were prepared by taking 300 microliters of wine diluted in 100 mL of solution (0.3% v / v).

5. The method of claim 2 **comprising** constructing a calibration curve for red wine with fixed concentration of glucose (0.01 M) and variable concentration of fructose concentrations 0.01; 0.03; 0.06 and 0.09 g / L in 0.1 M NaCl medium, injecting 15 microliters of each solution in a NaCl / NaOH 0.1 M flow for detection, and thus obtain a current or amperometric signal.

6. The method of claim 5, further comprising injecting 15 microliters of sample of red wine diluted in 0.1M NaCl medium, which were prepared by taking 500 microliters of wine diluted in 100 mL of solution (0.5% v / v).

7. The method of claim 1, further **comprising** constructing a curve from the average of the current intensities associated with each solution of the calibration curve and graphing each average value based on the concentration in units of g / L in order to obtain the equation of a straight line, where the value of X is the concentration of the wine sample.

8. E method of claims 4 and 6, **comprising** calculating the value of the concentration of the wine sample by replacing the value of the current of the wine sample in the Y component of the equation of the line.

FIGURE 1

FIGURE 2A

**FIGURA 2B**

**FIGURE 3**

**FIGURA 4**

**FIGURE 5**

EP 2 957 916 A1

**FIGURE 6**

**FIGURE 7**

10

Europäisches
Patentamt
European
Patent Office
Office européen
des brevets

# EUROPEAN SEARCH REPORT

Application Number

EP 15 17 3211

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CASELLA I G ET AL: "Amperometric detection of sulfur-containing compounds in alkaline media", THE ANALYST, vol. 127, no. 5, 2 April 2002 (2002-04-02), pages 647-652, XP055224078, ISSN: 0003-2654, DOI: 10.1039/b111080m * page 647, right-hand column, paragraph 2 - page 651, right-hand column, paragraph 2 * | 1-8 | INV. G01N35/08 G01N33/14 G01N27/49 G01N27/416 |
| X | CORBO D ET AL: "Use of a copper electrode in alkaline medium as an amperometric sensor for sulphite in a flow-through configuration", ANALYTICAL AND BIOANALYTICAL CHEMISTRY, vol. 374, no. 3, 11 September 2002 (2002-09-11), pages 416-420, XP055224101, ISSN: 1618-2642, DOI: 10.1007/s00216-002-1504-7 * the whole document * | 1-8 | |
| X | PAIXÃO T R L C ET AL: "Amperometric determination of ethanol in beverages at copper electrodes in alkaline medium", ANALYTICA CHIMICA ACTA, vol. 472, no. 1-2, November 2002 (2002-11), pages 123-131, XP055224090, ISSN: 0003-2670, DOI: 10.1016/S0003-2670(02)00942-X * page 124, left-hand column, paragraph 3 - page 125, left-hand column, paragraph 1 * * page 128, left-hand column, paragraph 3 - page 130, left-hand column, paragraph 2 * | 1-8 | |

TECHNICAL FIELDS SEARCHED (IPC)

G01N

-/--

The present search report has been drawn up for all claims

| Place of search | Date of completion of the search | Examiner |
|---|---|---|
| Munich | 29 October 2015 | Johnson, Keith |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
............................................................................
& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 15 17 3211

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP H05 149918 A (SHIMADZU CORP) 15 June 1993 (1993-06-15) | 1 | |
| A | * abstract * * paragraph [0008] - paragraph [0021] * * figures * | 2-8 | |
| A | LUQUE DE CASTRO M D ET AL: "Analytical Methods in Wineries: Is It Time to Change?", FOOD REVIEWS INTERNATIONAL, vol. 21, no. 2, April 2005 (2005-04), pages 231-265, XP055224096, ISSN: 8755-9129, DOI: 10.1081/FRI-200051897 * abstract * | 1-8 | |

TECHNICAL FIELDS
SEARCHED      (IPC)

The present search report has been drawn up for all claims

| Place of search | Date of completion of the search | Examiner |
|---|---|---|
| Munich | 29 October 2015 | Johnson, Keith |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT**
**ON EUROPEAN PATENT APPLICATION NO.**                     EP 15 17 3211

This annex lists the patent family members relating to the patent documents cited in the above-mentioned European search report.
The members are as contained in the European Patent Office EDP file on
The European Patent Office is in no way liable for these particulars which are merely given for the purpose of information.

29-10-2015

| Patent document cited in search report | Publication date | Patent family member(s) | Publication date |
|---|---|---|---|
| JP H05149918    A | 15-06-1993 | JP      3099474  B2<br>JP    H05149918  A | 16-10-2000<br>15-06-1993 |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0805350 A
- GB 2335985 A
- CN 102323313
- CN 102879445
- CN 202177599